# EUROPEAN PATENT SPECIFICATION

(11) **EP 2 937 014 B1**
(45) Date of publication and mention of the grant of the patent: **06.02.2019**
(21) Application number: 13865168.2
(22) Date of filing: 17.12.2013
(51) Int. Cl.: A45D 44/22, A45D 44/00

(54) **MANUFACTURING METHOD FOR PACKAGING BODY**
HERSTELLUNGSVERFAHREN FÜR EINEN VERPACKUNGSKÖRPER
PROCÉDÉ DE FABRICATION D'UN CORPS D'EMBALLAGE

(30) Priority: 18.12.2012 JP 2012275949
(43) Date of publication of application: 28.10.2015
(73) Proprietor: Taiki Corp., Ltd., Miyakojima-ku Osaka-shi Osaka 534-0014 (JP)
(72) Inventor: KURIBAYASHI, Yoshimitsu, Osaka-shi, Osaka 533-0031 (JP); SADAI, Osamu, Osaka-shi, Osaka 533-0031 (JP)
(74) Representative: Zimmermann & Partner Patentanwälte mbB
(86) International application number: PCT/JP2013/083687
(87) International publication number: WO 2014/098055

(56) References cited:
- WO-A1-2007/123380
- JP-A- H0 648 917
- JP-A- S5 951 019
- JP-A- S6 355 022
- JP-A- H02 169 513
- JP-A- H04 294 721
- JP-A- 2009 298 429
- JP-A- 2009 298 429
- JP-A- 2011 015 706
- JP-A- 2011 015 706
- US-A1- 2005 244 482

## Description

### [Technical Field]

The present invention relates to a method of making a package for a skin drug solution holding body where a drug solution is impregnated into a base material.

### [Background Art]

This type of skin drug solution holding body includes a face mask where liquid cosmetics as a drug solution is impregnated into a sheet-form base material made of, e.g., a nonwoven fabric. The face mask is stored in packaging bag and sealed therein for distribution. Then, a face mask is used by attaching it onto a face after being taken from a packaging bag. However, dripping is likely to occur during use when the viscosity of liquid cosmetics is low. That is, it is important to impregnate liquid cosmetics of high viscosity into a base material so as to prevent dripping. However, it will be difficult to uniformly impregnate liquid cosmetics throughout a base material in case of using liquid cosmetics of high viscosity, and so-called impregnation unevenness, in which liquid cosmetics remains in part without being impregnated, is likely to occur.

As a device to suppress such impregnation unevenness, a two-layer structure, for example, has been proposed for a nonwoven fabric constituting a base material in PTL 1 mentioned below. Also, it has been proposed that a folded base material be packed after being further wound into a roll form in PTL 2 mentioned below. Impregnation unevenness is less likely to occur even with liquid cosmetics of high viscosity by such devices, but a way to completely suppress impregnation unevenness has been further pursued. WO 2007/123380 A1 relates to a mask pack containing powder and a manufacturing method thereof, in which a sheet adhered with the powder, such as loess, and impregnated with cosmetic massage cream, such as Coenzyme Q10, is attached to a user's face to provide a user with a cosmetic effect in a simple and convenient process. The mask pack which is attached to a user' s face for a skin protective care includes a sheet having openings corresponding to eyes and mouth and slits for preventing the sheet from being loose, powder applied onto front and rear surfaces of the sheet, and a cosmetic massage cream impregnated on the sheet.

### [Citation List]

### [Patent Literature]

[PTL 1] Japanese Unexamined Patent Application Publication No. 2004-2253
[PTL 2] Japanese Unexamined Patent Application Publication No. 2011-15706

### [Summary of Invention]

### [Technical Problem]

Therefore, the present invention has been made in view of the above conventional problems, and an object thereof is to provide a package for a skin drug solution holding body capable of further suppressing the impregnation unevenness of the drug solution and a method of making the same.

### [Solution to Problem]

For solving the above problems a method of making a package for a skin drug solution holding body according to claim 1 is provided.

The package for a skin drug solution holding body having such configuration is vacuum-sealed and is in a state where there is little air inside the packaging bag. That is, since air is removed from the interior of the packaging bag in vacuum-sealing, there is little air between the inner surface of the packaging bag and the base material and also air hardly exists inside the base material. Additionally, the drug solution spreads to every corner inside the packaging bag, and also permeates every corner inside the base material.

In particular, it is preferable that the base material is in a sheet form and the packaging bag is a flat bag. In this case, since the base material is in a sheet form, it may be used by simply being attached to skin. In addition, since the packaging bag is a flat bag with no gore or gusset, a drug solution is likely to spread to every corner of the packaging bag. It is noted that the flat bag is in a bag form having no gore or gusset, including a butt-seam bag in addition to a two-sided (two-sided sealed) bag that has a two-folded portion as a bottom and has both right and left sides sealed as well as a three-sided (three-sided sealed) bag that has the bottom and both right and left sides sealed. The butt-seam bag is in a bag form where the bottom and the center rear portion are sealed, and the center rear portion has a rear bonding portion where the films overlap each other in a butt-seam way.

Furthermore, the base material is stored in a packaging bag in a folded state. In this case, it is possible to make the base material compact and keep the packaging bag small. Also, it is possible to compress the base material by vacuum-sealing without forming wrinkles or the like, and possible to maintain its folded state. Further, the drug solution securely permeates the interior of the base material by vacuum-sealing even when the base material is in a folded state.

The method of manufacturing a package for a skin drug solution holding is that a base material for use by attaching it to skin and a drug solution of high viscosity to be impregnated into the base material are placed in a packaging bag, and that the packaging bag in which the base material and drug solution are placed is vacuum-sealed. It is noted that as a sequence of placing the base material and drug solution in a packaging bag, the base material or the drug solution may be placed first, or both may be placed at the same time.

According to the method of manufacturing the package for a skin drug solution holding body having such configuration, air is removed from the interior of the packaging bag during vacuum-sealing, and, in the resulting vacuum-sealed state, a drug solution of high viscosity spreads throughout the interior of the packaging bag and at the same time permeates the interior of the base material. Also, even in a distribution process after manufacture, the package is continuously pressed from outside with air pressure (ordinary pressure), so that it is possible to further suppress the impregnation unevenness of a drug solution and to easily maintain such a state.

It is particularly preferable that a chamber-type vacuum sealing machine is used for vacuum-sealing. In case of the chamber type, a packaging bag is placed into a chamber, and the interior of the chamber is deaerated into a vacuum state. Then, an opening of the packaging bag is heat-sealed when the bag is in a predetermined vacuum state. The interior of the chamber is returned to ordinary pressure after being heat-sealed. While the interior of the chamber is returned to the ordinary pressure, the packaging bag is pressed from the outside with air pressure, so that the drug solution further permeates the interior of the base material with the pressing force.

Further, it is preferable to press, while sandwiching, the packaging bag containing the base material and drug solution therein prior to vacuum-sealing. In this case, it is possible to discharge excess air from the packaging bag in advance prior to vacuum-sealing, so that the base material and drug solution may be vacuum-sealed efficiently.

It is also preferable to press, while sandwiching, the package after vacuum-sealing. Because there remains little air inside the packaging bag, it is unnecessary to press the package strongly, and it is sufficient to sandwich the package with a small force. Accordingly, wrinkles formed on a packaging bag with pressing force are also prevented. Then, the drug solution further permeates the base material by pressing the package.

### [Advantageous Effects of Invention]

As described above, it is possible to suppress the impregnation unevenness of a drug solution more than ever before by vacuum-sealing in the present invention. Further, the amount of a drug solution for use may be small, and the volume of the package may also be reduced.

### [Brief Description of Drawings]

Fig. 1 is a front view of a face mask package as a package for a skin drug solution holding body made according to an embodiment of the present invention.
Fig. 2 is a front view as seen from the front side of an expanded state of a base material in the face mask package.
Figs. 3a, 3b, and 3c are front views showing a step of folding the base material.
Figs. 4a, 4b, 4c, and 4d are front views showing a step of folding the base material.
Fig. 5 is a front view showing a step of bagging the base material.
Fig. 6 is a cross-sectional view showing a pressing step prior to vacuum-sealing as one manufacturing step for the face mask package.
Fig. 7 is a cross-sectional view showing a vacuum-sealing step as one manufacturing step for the face mask package.
Fig. 8 is a front view showing a pressing step after vacuum-sealing as one manufacturing step for the face mask package.

### [Description of Embodiments]

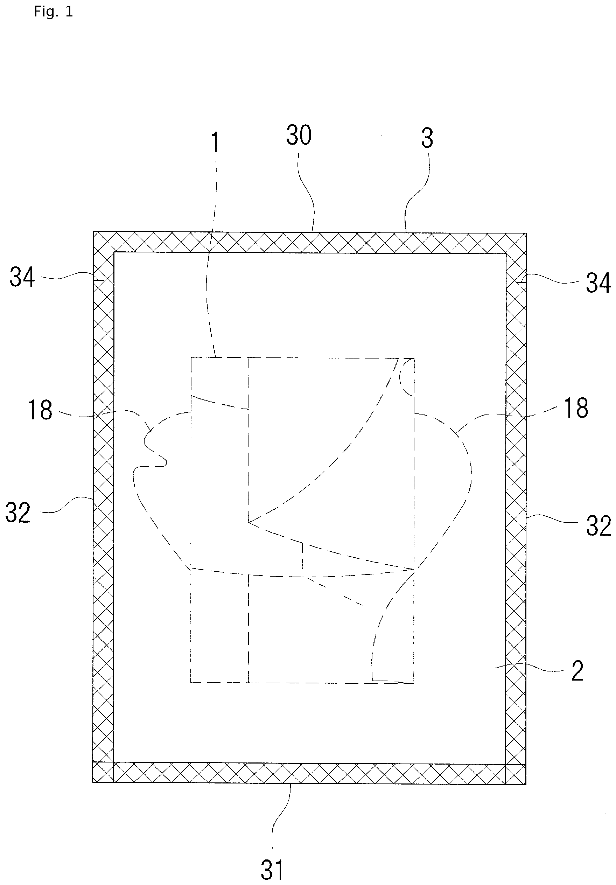

A package for a skin drug solution holding body and a method of making the same according to the present invention will be described below with reference to Fig. 1 to Fig. 8. The package for a skin drug solution holding body shown in Fig. 1 is a face mask package in which a face mask as a skin drug solution holding body is vacuum-sealed. The face mask is the one in which liquid cosmetics 2 as a drug solution is impregnated into a sheet-form base material 1 in use by attaching it to a face.

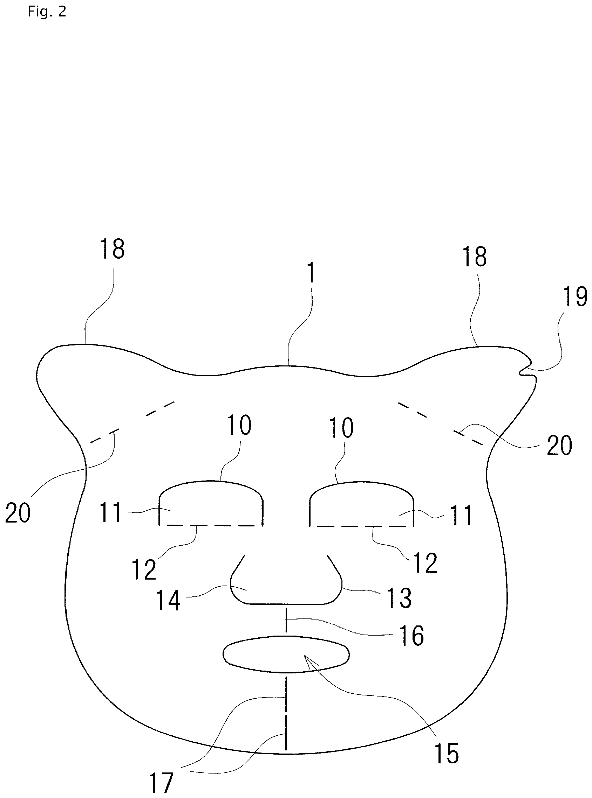

The base material 1 is stored in a packaging bag 3 in a folded state. It is noted that the way of folding the base material 1 will be described later. The base material 1 is formed in a face shape so as to cover almost an entire face as shown in Fig. 2. Fig. 2 is a development view obtained when the base material 1 is seen from the front side thereof, and the front side of the base material 1 is outside when the material is attached to a face. At locations corresponding to both eyes, a U-shaped cut line 10 for opening downward is formed respectively. Upward tongue piece-shaped eyelid-piece portion 11 is formed to be partitioned from other portions with the U-shaped cut line 10. There formed is a fold line 12 extending transversely at the base, in other words, at the lower end of the eyelid-piece portion 11. In a state where the material is stored in the packaging bag 3, both eyelid-piece portions 11 are folded back toward the front respectively so as to form a front valley fold along the fold line 12. Therefore, the side on the folded eyelid-piece portion 11 is the front side, so that the front and reverse of the base material may be easily distinguished thereby. It is noted that various fold ruled lines are applicable to the fold line 12, and may be, for example, a perforation line or a press-ruled line. Further, a U-shaped cut line 13 for opening upward is formed at a location corresponding to a nose, and a downward tongue piece-shaped nose-piece portion 14 is formed in a nose shape so as to be partitioned from other portions by the U-shaped cut line 13. Also, at a location corresponding to a mouth, a mouth opening 15 is formed in a horizontally long elliptic shape. Further, a vertical cut line 16 is formed between the nose cut line 13 and the mouth opening 15; a vertical cut line 17 is formed between the mouth opening 15 and the center of the outer edge lower end of the base material 1. By forming the vertical cut lines 16, 17 as such, the base material 1 is capable of easily fitting to an unevenly shaped face, and the base material 1 is likely to stick to the face.

Further, at the right and left corners of the upper end of the base material 1, knobs 18 projecting obliquely upward are formed respectively. The knobs 18 may be picked when the folded base material 1 is expanded as described later, so that the folded base material 1 may be easily expanded. Further, the base material 1 has a horizontally asymmetrical shape. Specifically, a cutout portion 19 is formed at the tip of the knob 18 on the right side seen from the front side (left side of a face when the material is adhered to a face), and the cutout portion is not formed at the knob 18 on the left side seen from the front side. Thus, as the material is attached to a face with picking the knob 18 on the right side as seen from the front side with a left hand, the base material 1 may be attached to a face without mistaking the front and reverse. Additionally, a fold line 20 is formed at the base of the knob 18. When the knob 18 is folded back outward and overlapped so as to form a front valley fold along the fold line 20 during use, the tip of the knob 18 is positioned at a location corresponding to the outer corner of the eye. Therefore, the base material 1 is made double at the outer corner of the eye, so that the liquid cosmetics 2 may be supplied in concentration mainly at the outer corner of the eye.

As described above, the base material 1 is in a sheet form. The sheet body constituting the base material 1 may be any material capable of absorbing and retaining liquid, but fiber sheets are particularly preferred and nonwoven fabrics are particularly preferable among them. For nonwoven fabrics, various materials may be used. It is possible to use nonwoven fabrics made of at least one fiber selected from the group consisting of, for example, polyolefin fibers, cellulose fibers, nylon fibers, polyester fibers, acrylic fibers, polyacrylic acid fibers, polylactic acid fibers, and polyurethane fibers, and nonwoven fabrics containing cellulose fibers are particularly preferred. Further, its weight per area (basis weight) is preferably 20 to 120 g/m², or more preferably 30 to 100 g/m².

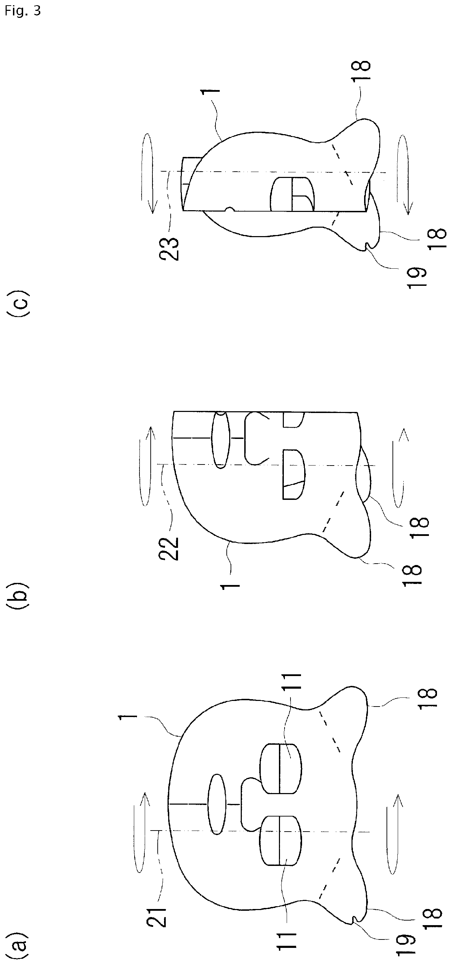

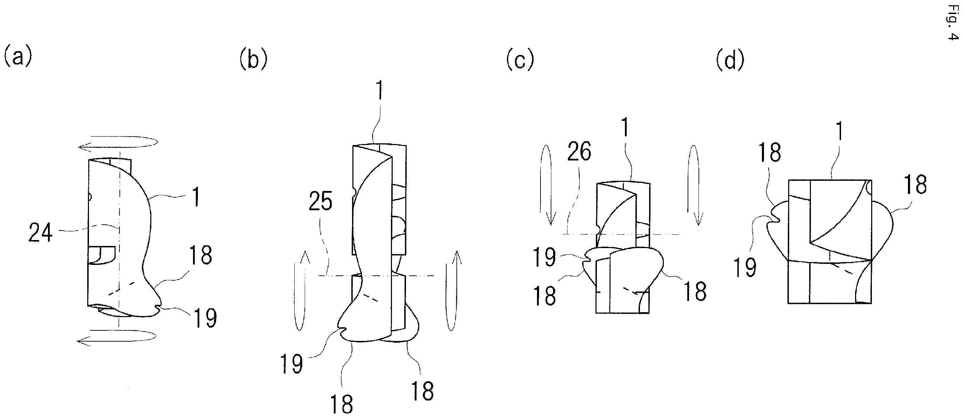

Such base material 1 is stored in the packaging bag 3 in a folded state. The material may be folded in any way, but an example thereof is shown in Fig. 3 and Fig. 4. It is noted that, in Fig. 3 and Fig. 4, the base material 1 is shown in a state of being turned upside down, it is merely an example and is not limited to this.

First, the both eyelid-piece portions 11 are folded respectively so as to form a front valley fold along the fold lines 12 as shown in Fig. 3(a). Fig. 3(a) shows a state viewed from the front side of the base material 1. Then, as a vertical imaginary folding line 21 is shown with a two-dot chain line, one of right and left sides is folded into a valley fold towards the other side at the side edge of a nose. Subsequently, as in Fig. 3(b), the base material 1 is reversed to the opposite side. Similarly, the opposite side is folded into a valley fold along a vertical imaginary folding line 22 shown with a two-dot chain line, thus folding it into a three-fold Z-shape from side to side. Then, as in Fig. 3(c), cheek areas on the sheet piece folded upward are folded into a valley fold along a vertical imaginary folding line 23 shown with a two-dot chain line. Next, as shown in Fig. 4(a), the material is reversed to the opposite side. The cheek areas on the opposite side are similarly folded into a valley fold along a vertical imaginary folding line 24 shown with a two-dot chain line. Subsequently, as shown in Fig. 4(b), the material is folded into a two-fold valley at a position above the eyes, that is, along a horizontal imaginary folding line 25 shown with a two-dot chain line. Then, as shown in Fig. 4(c), the material is folded into a valley fold at a position above the mouth, that is, along a horizontal imaginary folding line 26 shown with a two-dot chain line. By the folding step described above, the folded base material 1 is achieved as shown in Fig. 4 (d). Thus, the base material 1 is folded in a rectangular shape; in particular, a slightly elongated rectangular shape as a whole. In its folded state, the tips of the both knobs 18 protrude right and left respectively. Thus, the base material 1 may be easily expanded by pinching the both knobs 18 with both hands during use.

Now, the liquid cosmetics 2 injected to the packaging bag 3 will be explained. The liquid cosmetics 2 includes cosmetic liquid lotion, lotion, cosmetic liquid, emulsion, and the like, may be water- or oil-based, and is effective to skin, in particular, to face skin. For the liquid cosmetics 2, liquid cosmetics having a viscosity between 5,000 mPa·sec and 50,000 mPa·sec are used, so that the base material 1 is prevented from dripping during use. Viscosity adjustment may be carried out by various methods. The cosmetics is in a cream, paste, or gel form by viscosity adjustment.

There are various methods for viscosity adjustment, for example, a method using various types of thickeners, a method without the use of general thickeners. As for the thickeners, it is possible to use water-soluble and oil-based thickeners. As the water-soluble thickeners, any of natural water-soluble polymers, semisynthetic water-soluble polymers, synthetic water-soluble polymers, and the like may be used. The natural water-soluble polymers include, for example, xanthan gum, locust bean gum, succinoglucan, guar gum, carrageenan, gum arabic, tragacanth gum, pectin, and the like. The semisynthetic water-soluble polymers include, for example, cellulose-based polymers such as methyl cellulose, ethyl cellulose, hydroxyethyl cellulose, carboxymethyl cellulose, and the salts thereof (e.g., sodium salts and potassium salts); starch-based polymers such as soluble starch, carboxymethyl starch, and methyl starch; alginic acid-based polymers such as propylene glycol alginate and alginates; and polysaccharide-based derivatives. Synthetic water-soluble polymers include, for example, polyvinyl alcohol, polyvinyl pyrrolidone, polyvinyl methyl ether, carboxy vinyl polymers, acrylic acid-alkyl methacrylate copolymer, polyacrylic acid salts (e.g., sodium salts, potassium salts, and ammonium salts), polyethylene oxide, and ethylene oxide-propylene oxide block copolymer. The oil-based types include, for example, hydrocarbon-based waxes such as polyethylene wax, paraffin wax, synthetic hydrocarbon wax, microcrystalline wax, and ceresin wax; natural waxes such as carnauba wax, beeswax, lanolin wax, and candelilla; silicone waxes such as stearyl-modified methylpolysiloxane and behenyl-modified methylpolysiloxane; dextrin fatty acid ester such as dextrin palmitate, stearic acid dextrin, and isostearic acid dextrin; metal soaps such as isostearic acid aluminum, hydroxy stearic acid, and calcium stearate; organically-modified clay minerals; and oily gelling agents such as 12-hydroxy stearic acid. It is noted that the thickeners may be used alone or in combination of two or more thereof. Furthermore, the viscosity of the liquid cosmetics 2 may be measured by using, for example, BM viscometer (from TOKIMEC Inc.), and may be measured by using Rotor No. 4 at 12 rpm and 25°C for 1 min. However, measurement is carried out with appropriately adjusting a rotor in use and speed conditions based on a viscosity level.

In addition, the injection volume of the liquid cosmetics 2 to the packaging bag 3 is adjusted based on viscosity and application, the size of the base material 1, the size of the packaging bag 3, etc. The volume is, for example, about 5 to 20 times as much as the weight of dry base material 1. That is, impregnation magnitude in impregnating the liquid cosmetics 2 to a nonwoven fabric may be appropriately adjusted within the range of, e.g., 5 to 20 times. Here, the impregnation magnitude are calculated based on how many times liquid cosmetics 2 are used relative to the weight of a nonwoven fabric to be used. For example, in case of using 10 g of the liquid cosmetics 2 relative to 1 g of a nonwoven fabric, impregnation magnitude is 10 times.

Since the face mask package is vacuum-sealed, the liquid cosmetics 2 is spread throughout the interior of the packaging bag 3 and there remains little air inside the packaging bag 3. Even if air remains, the amount is slight. It is noted that although air remains inside the packaging bag 3, there remains no air inside the base material 1 and the air exists only slightly as air bubbles in the liquid cosmetics 2. The residual air ratio inside the packaging bag 3 are preferably 5% or less, or more preferably 2% or less. The residual air ratio may be measured by, e.g., the following method. First, a vacuum-sealed package is opened in water, and the air remaining in the package bag 3 is measured by collecting the air into an appliance for measuring volume, such as a graduated cylinder, and the amount is recorded as "capacity 1". Then, air is filled up in an empty packaging bag 3 of the same type and size. The air is released in water, and the air amount is similarly measured by collecting the air into an appliance for measuring volume, such as a graduated cylinder, and the amount is recorded as "capacity 2". A residual air ratio is determined from the calculating formula of residual air ratio (%) = "capacity 1"/"capacity 2" × 100.

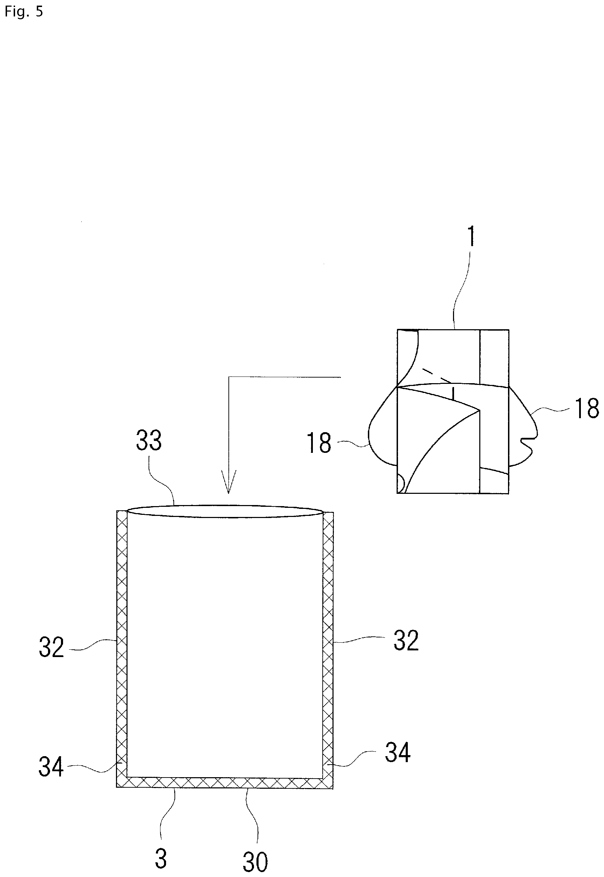

The packaging bag 3 is made of various types of flexible film. Various types of synthetic resin film may be used for a film with flexibility. The synthetic resin may be one or more kinds of resins selected from the group consisting of, e.g., polyamide resin, polyethylene resin, polyurethane resin, polystyrene resin, polypropylene resin, polyethylene terephthalate resin, nylon resin, polytrimethylene terephthalate resin, polylactic acid resin, polychlorotrifluoroethylene (PCTFE) resin, tetrafluoroethylene-hexafluoropropylene copolymer (FEP) resin, and ethylene-vinyl acetate copolymer resin. The film may be either a single layer or a multilayer, but a laminate film (multilayer film) having a heat seal layer for a layer constituting the inner surface is particularly preferred. Further, in order to improve the barrier properties of the packaging bag 3, a film with a deposit of aluminum or silica may be preferably used for the synthetic resin films. A laminated film having a metal foil such as aluminum in the middle may be preferably used. The thickness of the film is preferably 30 to 120 µm, or more preferably 50 to 80 µm. In order to retain liquid-tightness or the like, the width of the sealing portion is preferably about 3 to 20 mm or more preferably within the range of 5 to 20 mm at an insertion opening 33 of the packaging bag 3 (see Fig. 5). It is suitable to set the heating time during heat sealing within the range of about 1 to 5 seconds from the viewpoint of sealing property, liquid-tightness, etc., or more preferably from about 1 to 3 seconds. Further, it is possible, in the view of liquid-tightness and the like, to perform heat-sealing after applying an adhesive and the like to a portion to be sealed. It is also possible to seal the insertion opening 33 by using an ultrasonic bonding apparatus.

The packaging bag 3 may be of any bag form, but is a flat bag in this embodiment. The size of the packaging bag 3 may also be various depending on the size of the base material 1 therein. In this embodiment, the bag is in a vertically long rectangular shape, e.g., with the vertical dimension of about 150 mm and the transverse dimension of 100 mm or so. The periphery of the packaging bag 3 is heat-sealed over its entire circumference. The cross-hatched portion in Fig. 1 is a heat-sealed sealed portion. That is, all of the four sides on both upper and lower ends 30 and 31 and both right and left sides 32 are also heat-sealed at a predetermined width. It is noted that a so-called three-sided sealed flat bag is used for the packaging bag 3, and any one of an upper end 30 and a lower end 31 is used as the insertion opening 33 (see Fig. 5). A three-sided sealed flat bag having the insertion opening 33 at its lower end 31 is used in this embodiment. The insertion opening 33 at the lower end 31 is heat-sealed during vacuum-sealing. It is noted that notches 34 for opening are formed at both upper edges respectively. However, the notch 34 for opening may be provided on the side of the insertion opening 33.

Subsequently, steps of manufacturing the face mask package will be explained. The manufacturing steps mainly include steps of folding the base material 1, bagging the folded base material 1, injecting the liquid cosmetics 2, pressing the package prior to vacuum-sealing, vacuum-sealing, and pressing the package after vacuum-sealing. The step of folding the base material 1 is as described above. The bagging step is followed thereafter. The folded base material 1 as shown in Fig. 4 (d) is turned upside down. The lower end 31 is used as the insertion opening 33 as shown in Fig. 5, and the material is packed in the packaging bag 3 that is a three-sided sealed flat bag having the upper end 30 as its bottom. The injection step will follow thereafter. A predetermined amount of the liquid cosmetics 2 is poured from the insertion opening 33 into the interior of the packaging bag 3 in which the folded base material 1 is placed. It is noted that it is preferable to pour the liquid cosmetics 2 in gaps between the sheet pieces of the folded base material 1 themselves.

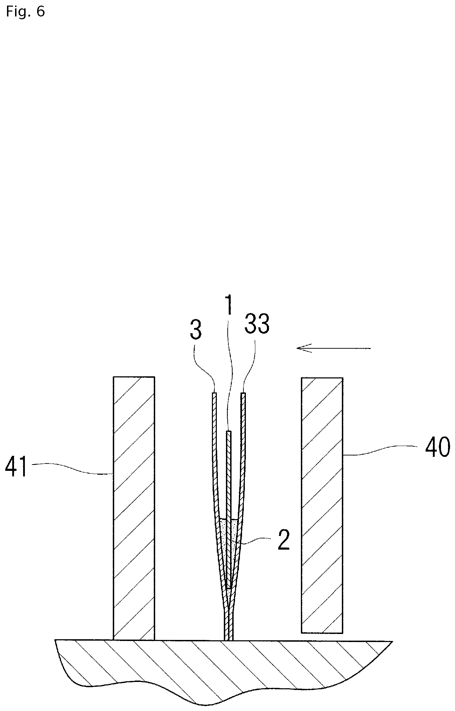

After the injection step, the pressing step prior to vacuum-sealing will follow. In the pressing step prior to the vacuum-sealing, the packaging bag 3 is placed, for example, between a pair of standing pressing walls 40, 41 as shown in Fig. 6. The inner surfaces of the pressing walls 40, 41 are flat. The packaging bag 3 has its insertion opening 33 faced up in an upright position. One of a pair of pressing walls 40, 41 is movable, and the other is fixed. The movable pressing wall 40 is reciprocally movable so as to move close or away from the fixed pressing wall 41. By approaching the movable pressing wall 40 towards the fixed pressing wall 41, the entire packaging bag 3 is pressed in the thickness direction (front-back direction) thereof. It is noted that the packaging bag 3 is sandwiched with both pressing walls 40, 41 for a predetermined period, for example, several seconds, and then the movable pressing wall 40 is separated. Excess air is removed from inside the packing bag 3 in advance by such a pressing step prior to vacuum-sealing. Additionally, it is also possible to apply the liquid cosmetics 2 to the base material 1 in advance by sandwiching the packaging bag 3 with the pressing walls 40, 41.

A chamber-type vacuum sealing machine is used in the vacuum-sealing step. Vacuum-sealing was performed by a vacuum sealing machine with the maximum degree of vacuum-sealing of -0.1 MPa (ordinary pressure basis) in this embodiment. It is possible to appropriately adjust the time for vacuum-sealing by using a vacuum sealing machine, based on the size or the like of the packaging bag 3. For example, a package may be completed by setting the packaging bag 3 in a vacuum sealing machine, then vacuum-sealing the bag for 10 to 120 seconds, and subsequently returning the bag to ordinary pressure. The time for the vacuum-sealing process is appropriately adjusted based on the size or the like of the packaging bag 3.

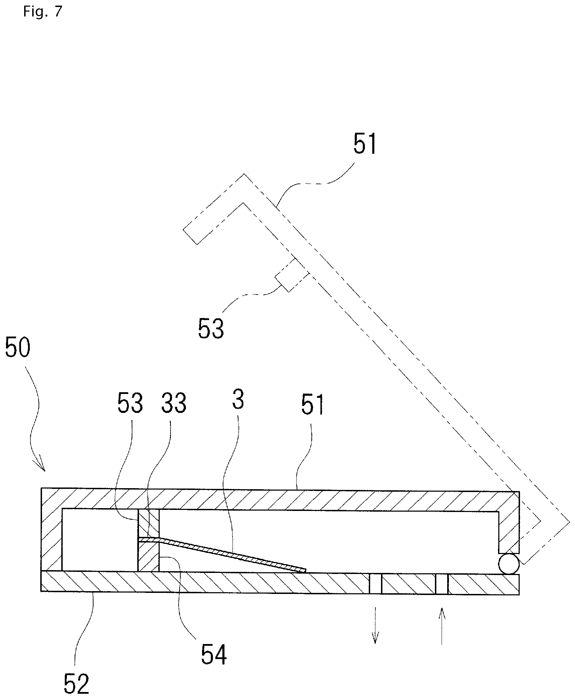

Specifically, an upper lid 51 of the chamber 50 is opened by being pivoted upward as shown with a two-dot chain line in Fig. 7, and the packaging bag 3 is placed in the chamber 50. A pair of laterally extending upper and lower seal bars 53, 54 are provided in the chamber 50. The upper seal bar 53 is secured to the inner surface of the upper lid 51 of the chamber 50. The upper seal bar 53 is a pressing seal bar having no built-in seal line. The lower seal bar 54 is fixed on a table 52 inside the chamber 50. The lower seal bar 54 is a heating seal bar having a built-in seal line. Then, the packaging bag 3 is placed on the table 52 so as to place the insertion opening 33 side of the packaging bag 3 on the lower seal bar 54. The upper surface of the lower seal bar 54 is higher than the upper surface of the table 52, so that the packaging bag 3 is inclined with its insertion opening 33 being upward. It is noted that the packaging bag 3 may be kept in an upright position, and the packaging bag 3 is placed in any manner. In any case, the bag is held so as not to spill the liquid cosmetics 2 as its content.

Then, the upper lid 51 of the chamber 50 is closed. By closing the upper lid 51 of the chamber 50, a pair of upper and lower seal bars 53, 54 vertically sandwich the insertion opening 33 of the packaging bag 3. Thereafter, air inside the chamber 50 is deaerated to decompress the chamber 50. When the interior of the chamber 50 has a predetermined vacuum condition, the insertion opening 33 is heat-sealed to be sealed. Pressure inside the chamber 50 is set at, e.g., -0.1 Mpa (ordinary pressure standard) of gauge pressure. After completing heat sealing, air is supplied into the chamber 50, and the pressure inside the chamber 50 is returned to ordinary pressure. The upper lid 51 of the chamber 50 is opened to take out a finished face mask package from the chamber 50. It is noted that the packaging bags 3 may be vacuum-sealed individually, but it is preferable to vacuum-seal multiple bags simultaneously. For example, when multiple packaging bags 3 are laterally arranged with a gap therebetween along the lower seal bar 54, it is possible to vacuum-seal the bags efficiently.

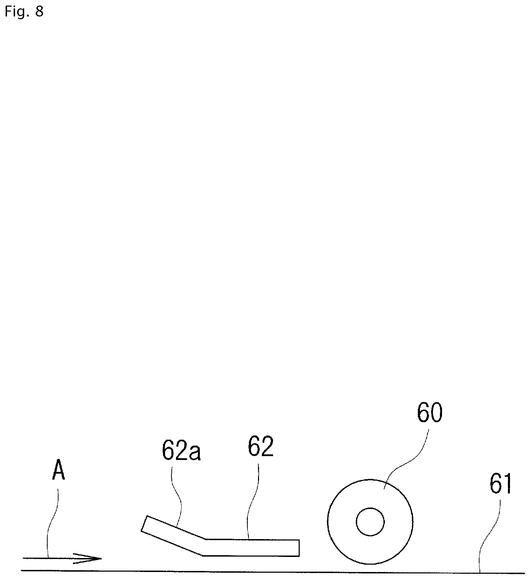

It is preferable to perform the pressing step after the vacuum-sealing as described above. That is, the completed package is pressed, as if to be sandwiched, in the thickness direction thereof. The pressing means can be any, but a roller 60 shown in Fig. 8, for example, may be used. In case of using the roller 60, the rollers 60 rotating with a horizontal axis, for example, is arranged above a belt conveyor 61 with a gap therebetween, and the package is placed on the belt conveyor 61 in a laid-down position and is carried to pass under the roller 60. A symbol A indicates a conveyance direction in Fig. 8. By carrying the package on the belt conveyor 61 in this manner, the package may be pressed by vertically sandwiching the package between the roller 60 and the belt conveyor 61. In this case, the orientation of the package may be either vertical or horizontal. Also, it is preferable to arrange a guide plate 62 on the upstream side of the roller 60. The guide plate 62 is arranged above the belt conveyor 61 with a gap therebetween, and the package is pressed from the above with the guide plate 62, so that the thickness of the package may be made uniform. By arranging the guide plate 62 on the upstream side of the roller 60 in this manner, wrinkles are prevented from being formed on the packaging bag 3 when the package is pressed by the roller 60. It is noted that it is preferable to make the height of the roller 60 and the guide plate 62 adjustable. It is possible to adjust a gap between the belt conveyor 61 and the roller 60 and a gap between the belt conveyor 61 and the guide plate 62. The gap between the belt conveyor 61 and the guide plate 62 is set larger than the gap between the belt conveyor 61 and the roller 60. Further, it is preferable to upwardly incline an upstream end 62a of the guide plate 62 so as to make a gap with the belt conveyor 61 increase gradually towards the upstream. With this configuration, the package smoothly goes under the guide plate 62. It is noted that the number of the rollers 60 may be either single or more than one. Further, the roller 60 may be arranged in a vertical, instead of horizontal, direction. That is, in the upright position, the package may pass through between a pair of the rollers 60 rotating with a vertical axis. In any case, by pressing, as if to sandwich, the package in the thickness direction after vacuum-sealing, the liquid cosmetics 2 permeates the base material 1 more evenly. It is also sufficient to press with small force since there remains little air in the packaging bag 3, so that wrinkles are prevented from being formed on the packaging bag 3. Moreover, since the thickness of the packages is made uniform, in case of packing multiple packages into a box or the like, it is possible to box packages smoothly and also to place many packages.

As described above, according to the face mask package in this embodiment, there is little air inside the packaging bag 3 by vacuum-sealing; the liquid cosmetics 2 spreads to every corner inside the packaging bag 3; and the liquid cosmetics 2 permeates every corner inside the base material 1. Therefore, it is certainly possible to prevent impregnation unevenness from occurring even when the liquid cosmetics 2 of high viscosity is used. Furthermore, the injection volume of the liquid cosmetics 2 can be small, and the volume of a face mask package may be reduced.

Also, because the folded base material 1 is stored in the packaging bag 3, the base material 1 can be compacted and the size of the packaging bag 3 is also sufficiently small. Moreover, wrinkles or the like are not formed on the base material 1 even if the material is compressed by vacuum-sealing. Further, the liquid cosmetics 2 permeates securely inside the base material 1 by vacuum-sealing even if the base material 1 is in a folded state, so that uneven impregnation may be prevented. Also, since the packaging bag 3 is in the form of a flat bag with no gore or gusset, the liquid cosmetics 2 is likely to spread to every corner of the packaging bag 3. Further, the knob 18 is formed on the base material 1, so that the base material 1 may be easily expanded even if the liquid cosmetics 2 of high viscosity is used.

Furthermore, since excess air is removed from inside the packaging bag 3 by providing the pressing step prior to vacuum-sealing and sandwiching the packaging bag 3 with the pressing walls 40, 41, the liquid cosmetics 2 is applied well to the base material 1. Furthermore, the interior of the chamber 50 is returned to ordinary pressure after heat sealing, so that the sealed packaging bag 3 is pressed by air pressure, and the liquid cosmetics 2 permeates further with the pressing force.

In addition, one detailed specific example will be described below. A nonwoven fabric of a basis weight of 70 g/m², including rayon fiber (40%)/polyester and polyethylene splittable fiber (60%), is used as a base material 1. The base material 1 may be folded as described above, but the material is folded into, for example, a three-fold of a horizontally Z-shape and then folded further into a two-fold in the vertical direction, thus being folded into the total of six folds. "SIMUL GEL NS" from SEPPIC Corporation is used as a thickener. The packaging bag 3 has a three-sided sealed structure using a film of a three-layer structure. This film of the three-layer structure is made of, in order from the surface, 12 µm-thick polyethylene terephthalate film, 9 µm-thick aluminum foil, and 40 µm-thick polypropylene film. The viscosities of the liquid cosmetics 2 are adjusted to 5,000 mPa·sec, 10,000 mPa·sec, 20,000 mPa·sec, 30,000 mPa·sec, 40,000 mPa ·sec, and 50,000 mPa ·sec respectively, and the impregnation magnification is also about 10 to 13 times. As a vacuum sealing machine, Model TVS-8500B from Nishihara Manufacturing Co., Ltd. is used to vacuum-seal the material by reducing pressure for about 15 to 20 seconds (maximum: -0.1 Mpa). Good impregnation state was confirmed at any viscosity.

It is noted that, in the above description, a chamber-type vacuum sealing machine is used, but a nozzle vacuum sealing machine may also be used.

Further, the packaging bag 3 is pressed with the pressing walls 40, 41, prior to vacuum-sealing; however, the pressing means is not limited to the pressing walls 40, 41, and the bag may be sandwiched with a pair of rollers, for example. Further, the pressing step prior to the vacuum-sealing may be omitted. Similarly, the pressing step after vacuum-sealing is optional and may be omitted.

Furthermore, the method of folding the base material 1 is optional. Besides the one described above, various ways such as six-fold and 12-fold may be used. In addition to storing the material in the packaging bag 3 in a folded state, it may be stored in the packaging bag 3, for example, in a rolled-up state. In this case, it may be stored in the packaging bag 3 by, for example, folding the base material 1 into a six-fold or the like and then further winding it into a roll-shape. The base material 1 in a flat form without folding or winding it into a roll shape may be stored in the packaging bag 3. For example, in case of a point mask (point facial pack) or a separate mask having a size enough to cover a part of a face, the base material 1 in a flat form without folding may be stored in the packaging bag 3.

Also, besides using a three-sided sealed flat bag as the packaging bag 3, it is possible to use a flat bag, such as a butt-seam bag where the bottom and the center rear portion (rear bonding portion) are sealed. Furthermore, instead of a flat bag, it may be in a form of a bag with a gore or gusset.

Further, in order to easily find the front and reverse of the base material 1, a cutout portion 19 is formed at one of the knobs 18 to make the base material 1 asymmetrical in its shape. By differentiating the shape right and left, except for the knobs 18, the base material 1 may also be shaped asymmetrical. However, the base material 1 may have undistinguishable front and reverse. In that case, the base material 1 may be horizontally asymmetrical or may be horizontally symmetrical.

The base material 1 made of a nonwoven fabric was explained, but the material may also be made of fibrous material such as a woven fabric and cosmetic cotton and may be porous, such as a sponge. In any case, it is preferable, as long as the material is capable of absorbing and retain liquid, but in particular, a thin material is preferable since it easily adheres to skin, so that the material may be, for example, a thinly sliced sponge. It is noted that In the case of cosmetic cotton, the one with 120 to 250 g/m² in its basis weight may be appropriately used. Further, the impregnation magnitude in impregnating the liquid cosmetics 2 in cosmetic cotton may be appropriately adjusted within the range of 10 to 30 times. Also, in case of a sponge, it is possible to appropriately use the one which is molded with various materials (e.g., NBR and NR). Moreover, in addition to the one for covering almost an entire face, the base material 1 may be the one for partially covering a face, such as a point facial pack and a separate mask. Further, it may also be used for skin other than a face. Also, in addition to the one in which the base material 1 keeps of adhering to skin even when released by hands, it may be a type in which the base material 1 needs to be continuously pressed onto skin, or may be one in which the base material 1 is attached on skin only for a short time.

It is noted that although the case of using the liquid cosmetics 2 as a drug solution is described, the drug solution is not limited to this and may be a liquid medicine having medicinal properties, a quasi-drug or the like.

### [Reference Signs List]

- 1: Base material
- 2: Liquid cosmetics
- 3: Packaging bag
- 10: Cut line
- 11: eyelid-piece portion
- 12: Fold line
- 13: Cut line
- 14: Nose-piece portion
- 15: Opening for mouth
- 16: Cut line
- 17: Cut line
- 18: Knob
- 19: Cutout portion
- 20: Fold line
- 21: Imaginary folding line
- 22: Imaginary folding line
- 23: Imaginary folding line
- 24: Imaginary folding line
- 25: Imaginary folding line
- 26: Imaginary folding line
- 30: Upper end
- 31: Lower end
- 32: Side
- 33: Insertion opening
- 34: Notch
- 40: Movable pressing wall
- 41: Fixed pressing wall
- 50: Chamber
- 51: Upper lid
- 52: Table
- 53: Upper seal bar
- 54: Lower seal bar
- 60: Roller
- 61: Belt conveyor
- 62: Guide plate
- 62a: Upstream end

## Claims

1. A method of making a package for a skin drug solution holding body comprising: placing a base material to be used by attaching it to skin and a drug solution for impregnating the base material, in a packaging bag; and
vacuum-sealing the packaging bag in which the base material and drug solution are placed,
wherein the drug solution has a viscosity of between 5,000 mPa · sec and 50,000 mPa · sec,
wherein the base material that is folded along a horizontal folding line, is placed in the packaging bag having an opening at any one of an upper end and a lower end, and
wherein while a drug solution is being injected from an opening of the packaging bag into the interior of the packaging bag in which a folded base material is placed, the drug solution is injected into gaps between sheet pieces of the folded base material.

2. The method of making a package for a skin drug solution holding body according to claim 1, wherein the packaging bag in which the base material and drug solution are placed, is put inside a chamber of a vacuum sealing machine and the interior of the chamber is brought into a vacuum state; an opening of the packaging bag is heat-sealed inside the chamber in a vacuum state so as to seal the opening of the packaging bag; and pressure inside the chamber is returned to ordinary pressure after the heat sealing.

3. The method of making a package for a skin drug solution holding body according to claim 2, wherein the opening of the packaging bag is sandwiched with a pair of seal bars inside the chamber, and then the interior of the chamber is brought into a vacuum state.

4. The method of making a package for a skin drug solution holding body according to claim 3, wherein a portion on an opening side of the packaging bag is placed on a lower seal bar inside the chamber and the packaging bag is inclined to have its opening upward; and then, an upper lid of the chamber is closed, so that the opening of the packaging bag is vertically sandwiched with an upper seal bar fixed to an inner surface of the upper lid and the lower seal bar.

5. The method of making a package for a skin drug solution holding body according to claim 1, wherein, prior to vacuum-sealing, the packaging bag in which the base material and drug solution are placed is pressed while being sandwiched.

6. The method of making a package for a skin drug solution holding body according to claim 5, wherein the package bag is in an upright position so as to face its opening upward, and the packaging bag is entirely sandwiched with a pair of pressing walls.

7. The method of making a package for a skin drug solution holding body according to claim 6, wherein the state of sandwiching the packaging bag with a pair of pressing walls is kept for a predetermined time.

8. The method of making a package for a skin drug solution holding body according to claim 1, wherein, after vacuum-sealing, a package is pressed while being sandwiched.

9. The method of making a package for a skin drug solution holding body according to claim 8, wherein the package is carried on a belt conveyor and is pressed by vertically sandwiching the package with a roller that is arranged above the belt conveyor, and the belt conveyor, and preferably wherein after the package is pressed from above with a guide plate arranged on an upstream of the roller, the package is further pressed by vertically sandwiching the package with the roller and the belt conveyor.

## Patentansprüche

1. Verfahren zum Herstellen einer Verpackung für einen Bereithaltungskörper einer Hautmedikamentenlösung, umfassend:
Anordnen eines Grundmaterials, das verwendet werden soll, indem es auf die Haut aufgelegt wird, und einer Medikamentenlösung zum Imprägnieren des Grundmaterials, in einem Verpackungsbeutel; und
Vakuumversiegeln des Verpackungsbeutels, in dem das Grundmaterial und die Medikamentenlösung angeordnet sind,
wobei die Medikamentenlösung eine Viskosität von zwischen 5.000 mPa · sec und 50.000 mPa · sec hat,
wobei das Grundmaterial, das entlang einer horizontalen Faltlinie gefaltet ist, in dem Verpackungsbeutel angeordnet wird, der eine Öffnung an einem oberen Ende und einem unteren Ende hat, und
wobei, während eine Medikamentenlösung von einer Öffnung des Verpackungsbeutels aus in das Innere des Verpackungsbeutels eingespritzt wird, in dem ein gefaltetes Grundmaterial angeordnet ist, die Medikamentenlösung in Spalten zwischen Schichtstücken des gefalteten Grundmaterials eingespritzt wird.

2. Verfahren zum Herstellen einer Verpackung für einen Bereithaltungskörper einer Hautmedikamentenlösung nach Anspruch 1, wobei der Verpackungsbeutel, in dem das Grundmaterial und die Medikamentenlösung angeordnet sind, in eine Kammer einer Vakuumverschließmaschine eingebracht wird, und das Innere der Kammer in einen Vakuumzustand versetzt wird; eine Öffnung des Verpackungsbeutels in der in einem Vakuumzustand befindlichen Kammer wärmeversiegelt wird, um die Öffnung des Verpackungsbeutels zu versiegeln; und ein Druck in der Kammer nach dem Wärmeversiegeln auf einen gewöhnlichen Druck rückgeführt wird.

3. Verfahren zum Herstellen einer Verpackung für einen Bereithaltungskörper einer Hautmedikamentenlösung nach Anspruch 2, wobei die Öffnung des Verpackungsbeutels mit einem Paar Siegelschienen in der Kammer eingeklemmt und dann das Innere der Kammer in einen Vakuumzustand versetzt wird.

4. Verfahren zum Herstellen einer Verpackung für einen Bereithaltungskörper einer Hautmedikamentenlösung nach Anspruch 3, wobei ein Teilbereich auf einer Öffnungsseite des Verpackungsbeutels an einer unteren Siegelschiene in der Kammer angeordnet und der Verpackungsbeutel geneigt wird, damit seine Öffnung nach oben gewandt ist; und dann ein oberer Deckel der Kammer geschlossen wird, so dass die Öffnung des Verpackungsbeutels mit einer oberen Siegelschiene, die an einer Innenfläche des oberen Deckels befestigt ist, und der unteren Siegelschiene eingeklemmt ist.

5. Verfahren zum Herstellen einer Verpackung für einen Bereithaltungskörper einer Hautmedikamentenlösung nach Anspruch 1, wobei vor dem Vakuumversiegeln der Verpackungskörper, in dem das Grundmaterial und die Medikamentenlösung angeordnet sind, gepresst und dabei eingeklemmt wird.

6. Verfahren zum Herstellen einer Verpackung für einen Bereithaltungskörper einer Hautmedikamentenlösung nach Anspruch 5, wobei sich der Verpackungsbeutel in einer aufrechten Position befindet, damit seine Öffnung nach oben gewandt ist, und der Verpackungsbeutel insgesamt mit einem Paar von Presswänden eingeklemmt wird.

7. Verfahren zum Herstellen einer Verpackung für einen Bereithaltungskörper einer Hautmedikamentenlösung nach Anspruch 6, wobei der Zustand, den Verpackungsbeutel mit einem Paar von Presswänden einzuklemmen, für eine vorbestimmte Zeit beibehalten wird.

8. Verfahren zum Herstellen einer Verpackung für einen Bereithaltungskörper einer Hautmedikamentenlösung nach Anspruch 1, wobei nach dem Vakuumversiegeln eine Verpackung gepresst und dabei eingeklemmt wird.

9. Verfahren zum Herstellen einer Verpackung für einen Bereithaltungskörper einer Hautmedikamentenlösung nach Anspruch 8, wobei die Verpackung auf einem Bandförderer transportiert und gepresst wird, indem die Verpackung mit einer über dem Bandförderer angeordneten Walze und dem Bandförderer vertikal einklemmt wird, und wobei vorzugsweise, nachdem die Verpackung von oben mit einer der Walze vorgelagerten Führungsplatte gepresst wurde, die Verpackung weiter gepresst wird, indem die Verpackung vertikal mit der Walze und dem Bandförderer eingeklemmt wird.

## Revendications

1. Procédé de fabrication d'un emballage pour un corps supportant une solution de médicament pour la peau comprenant : le placement d'un matériau de base à utiliser par fixation de celui-ci à de la peau et d'une solution de médicament pour une imprégnation du matériau de base, dans un sac d'emballage ; et
le scellement sous vide du sac d'emballage dans lequel le matériau de base et la solution de médicament sont placés,
dans lequel la solution de médicament présente une viscosité de 5 000 MPa.s à 50 000 MPa.s,
dans lequel le matériau de base qui est plié le long d'une ligne de pliage horizontal, est placé dans le sac d'emballage présentant une ouverture à l'une quelconque d'une extrémité supérieure et d'une extrémité inférieure, et
dans lequel alors qu'une solution de médicament est injectée à partir d'une ouverture du sac d'emballage dans l'intérieur du sac d'emballage dans lequel un matériau de base plié est placé, la solution de médicament est injectée dans des espaces entre des morceaux de feuilles du matériau de base plié.

2. Procédé de fabrication d'un emballage pour un corps supportant une solution de médicament pour la peau selon la revendication 1, dans lequel le sac d'emballage dans lequel le matériau de base et la solution de médicament sont placés, est déposé à l'intérieur d'une chambre d'une machine de scellement sous vide et l'intérieur de la chambre est mise dans un état de vide ; une ouverture du sac d'emballage est thermoscellée à l'intérieur de la chambre dans un état de vide afin de sceller l'ouverture du sac d'emballage ; et une pression à l'intérieur de la chambre est remise à la pression atmosphérique après le thermoscellement.

3. Procédé de fabrication d'un emballage pour un corps supportant une solution de médicament pour la peau selon la revendication 2, dans lequel l'ouverture du sac d'emballage est prise en sandwich avec une paire de barres de scellement à l'intérieur de la chambre, et l'intérieur de la chambre est ensuite mise dans un état de vide.

4. Procédé de fabrication d'un emballage pour un corps supportant une solution de médicament pour la peau selon la revendication 3, dans lequel une portion d'un côté d'ouverture du sac d'emballage est placée sur une barre de scellement inférieure à l'intérieur de la chambre et le sac d'emballage est incliné pour présenter son ouverture vers le haut ; et puis, un couvercle supérieur de la chambre est fermé, de sorte que l'ouverture du sac d'emballage est verticalement prise en sandwich avec une barre de scellement supérieure fixée à une surface interne du couvercle supérieur et la barre de scellement inférieure.

5. Procédé de fabrication d'un emballage pour un corps supportant une solution de médicament pour la peau selon la revendication 1, dans lequel, avant le scellement sous vide, le sac d'emballage dans lequel le matériau de base et la solution de médicament sont placés est pressé tout en étant pris en sandwich.

6. Procédé de fabrication d'un emballage pour un corps supportant une solution de médicament pour la peau selon la revendication 5, dans lequel le sac d'emballage est dans une position verticale afin de mettre son ouverture vers le haut, et le sac d'emballage est entièrement pris en sandwich avec une paire de parois de pressage.

7. Procédé de fabrication d'un emballage pour un corps supportant une solution de médicament pour la peau selon la revendication 6, dans lequel l'état de prise en sandwich du sac d'emballage avec une paire de parois de pressage est maintenu pendant une durée prédéterminée.

8. Procédé de fabrication d'un emballage pour un corps supportant une solution de médicament pour la peau selon la revendication 1, dans lequel, après le scellement sous vide, un emballage est pressé tout en étant pris en sandwich.

9. Procédé de fabrication d'un emballage pour un corps supportant une solution de médicament pour la peau selon la revendication 8, dans lequel l'emballage est supporté sur une courroie transporteuse et est pressé par prise en sandwich verticalement de l'emballage avec un rouleau qui est disposé au-dessus de la courroie transporteuse, et la courroie transporteuse, et de préférence dans lequel après que l'emballage est pressé à partir du dessus avec une plaque de guidage disposée en amont du rouleau, l'emballage est encore pressé par prise en sandwich verticalement de l'emballage avec le rouleau et la courroie transporteuse.
